# EUROPEAN PATENT APPLICATION

(11) **EP 3 838 284 A1**
(43) Date of publication of application: **23.06.2021**
(21) Application number: 20810850.6
(22) Date of filing: 30.10.2020
(51) Int. Cl.: A61K 36/537, A61K 33/30, A61K 31/343, A61P 13/08, A61P 17/14, A23L 33/105, A23L 33/16

(54) **COMPOSITION, COMPRISING SALVIA MILTIORRHIZA BUNGE EXTRACT AS ACTIVE INGREDIENT, FOR PREVENTION OR TREATMENT OF BENIGN PROSTATIC HYPERPLASIA OR ALOPECIA**

(30) Priority: 31.10.2019 KR 20190137533; 02.12.2019 KR 20190157928; 23.04.2020 KR 20200049163; 29.10.2020 KR 20200142540
(71) Applicant: Curomebioscience Co., Ltd., Jiksan-ro, Jiksan-eup, Seobuk-gu, Cheonan-si, Ch'ungch'ong namdo 31035 (KR)
(72) Inventor: YUN, Joo Seog, Gyeonggi-do 16707 (KR); SEO, Kang sik, Gyeonggi-do 15388 (KR); HAN, Jeong Su, Gyeonggi-do 16485 (KR); KIM, Eun Kyung, Busan 46765 (KR)
(74) Representative: Beckmann, Claus
(86) International application number: PCT/KR2020/015058
(87) International publication number: WO 2021/086120

(57) **Abstract**

The present disclosure relates to a compositoin comprising a *Salvia miltiorrhiza* Bunge extract as an active ingredient for treatment or prevention of benign prostatic hyperplasia or alopecia, the *Salvia miltiorrhiza* Bunge extract being characteized by containing tanshinones in 2-70%, including tanshinone1, tanshinone2a, cryptotanshinone, and dihydrotanshinone, and can provide a pharmaceutical compositoin and a helath fucntional food for treatment or prevention of alopecia and benign prostatic hyperplasia.

## Description

### Technical Field

The present invention relates to a composition containing a *Salvia miltiorrhiza* Bunge extract as an active ingredient for treatment or prevention of benign prostatic hyperplasia or alopecia.

### Background Art

Benign prostatic hyperplasia (BPH) is a common chronic disease of the urinary system among men over 50 years (Berry S.J. et al., 1984). BPH is also associated with lower urinary tract symptoms (LUTS) including increased urinary intermittency, increased urinary frequency, urinary urgency, weak urinary stream, and incomplete bladder emptying, and these degrade the quality of life of the elderly and have a negative effect on their daily lives (Sarma, A.V. et al., 2012).

Although the fundamental cause of BPH is unknown, it has been well established that the prostate gland in aging men is affected by androgens (Kim S.K. et al., 2015). During BPH, androgens promote the proliferation of epithelial cells or stromal cells in the prostate gland in an autocrine or paracrine manner, resulting in the imbalance in prostate cell proliferation and apoptosis. This has been considered to be an important cause of BPH (Choi, H.M. et al., 2016).

Especially, dihydrotestosterone (DHT) is well known to be associated with the development of BPH. With advancing years, testosterone, a male sex hormone, begins to be converted to dihydrotestosterone (DHT) at a high rate in the prostate, and this is primarily due to an increase in concentration of reductase, an enzyme that converts testosterone to DHT. DHT is a more potent androgen compared to TST because of its higher binding for the androgen receptor (AR). 5-α reductase type 2 (5AR2) converts TST to DHT in the prostate gland, and both DHT binds to AR to increase the transcription of androgen-dependent genes, ultimately causing the stimulation of protein synthesis associated with prostate proliferation and improving prostate-specific antigen (PSA) levels. The PSA levels increase during BPH and prostate cancer, and therefore, PSA is widely used for the diagnosis of BPH (Chen, Y. et al., 1996).

The cell cycle takes place in prostatic stromal and epithelial cells, leading to their division and duplication. The G1/S progression is highly regulated by cyclin D1. In addition, proliferating cell nuclear antigen (PCNA) is an acidic nuclear protein that has been recognized as a histological marker for the G1/S phase in the cell cycle. Therefore, the expression of PCNA and cyclin D1 can reflect the proliferation state of prostatic cells during BPH (Wang, W., et al., 2004).

On the contrary, apoptosis in the prostate epithelia occurs more frequently in the prostate under normal conditions than during BPH. Bcl-2, which inhibits apoptosis, is also found in the prostate epithelium, leading to the growth of the prostate tissue (Cardillo, M., et al., 1997).

One of other symptoms caused by male hormones, such as dihydrotestosterone (DHT), is male pattern baldness. The causes of hair loss have been presented to be poor blood circulation, excessive functioning of male sex hormones, excessive secretion of sebum, deterioration of scalp functions due to dandruff bacteria and other bacteria, genetic factors, aging stress, and the like, but the causes of androgenic alopecia, which is the most common type of hair loss directly associated with excessive secretion of male sex hormones, have been revealed until now.

During androgenic alopecia, healthy hairs gradually become thinner and shorter, and the hairs become weaker and thus break easily, and such a phenomenon is called miniaturization. The hair follicles undergoing miniaturization ultimately become thinner, invisible, and changed to short downy hair, eventually leading to hair loss. Therefore, many studies have recently been reported for the prevention and treatment of hair loss through inhibition of male hormone activity.

Testosterone is converted to dihydrotestosterone (DHT), an active male hormone, by 5α-reductase, and such activated dihydrotestosterone binds to the androgen receptor, thereby delaying the protein synthesis of hair follicular cells, resulting in shortage in growth period of hair follicles, and shrinking hair follicles, causing hair loss. In addition, excessive sebum is produced during androgenic hair loss, and as a result, hair loss accompanied by inflammation may appear in the scalp (Dennis A. Holt, et. al,. 1990).

The benign prostatic hypertrophy treatments used as alpha-1 adrenergic blockers, such as terazosin, doxazosin, tamsulosin, and alfuzosin, which have been currently developed, relax prostate smooth muscles to relieve urethral obstruction symptoms, but have been reported to cause side effects, such as hypotension and vasodilatory headache, due to the sympathetic block.

Finasteride (Fi) and dutasteride, which are 5α-reductase inhibitors, are known to reduce prostate sizes and be effectively used in BPH and alopecia by inhibiting the conversion of testosterone into the active metabolite dihydrotestosterone (DHT) through 5α reductase (Park, E.S., et al., 2018), and these drugs are known to cause side effects, such as erectile dysfunction, reduction of sexual desire, reduction in semen volume in ejaculation. As such, the currently used drug treatments are somewhat effective, but the side effects thereof are a problem, and actually, benign prostatic hyperplasia is one of persistent aging phenomena and is difficult to treat fundamentally.

Therefore, researchers looking for effective treatment strategies with fewer side effects are increasingly interested in alternative medicine including drugs manufactured of natural materials. Currently, only saw palmetto extracts are widely known as a functional food having an effect on BPH among natural materials (Marks, L.S., et al., 2000) .

*Salvia miltiorrhiza* Bunge is a perennial herb belonging to the Lamiaceae family, and is named *"Danshen"* since its roots are similar in shape to ginseng and has red color. *Salvia miltiorrhiza* Bunge has long been known as a medicinal herb that strengthens blood vessels, and it is known that the tanshinones contained in *Salvia miltiorrhiza* Bunge inhibit the oxidization of waste products in blood vessels to help rejuvenate blood vessels, expand blood vessels to improve blood circulation, and prevent a vascular disease, such as hypertension or myocardial infarction. It has also been recorded in Korean medicine that *Salvia miltiorrhiza* Bunge is a medicinal herb promoting blood flow and eliminating blood stagnation to help the creation of new blood.

Korean Patent Publication No. 2012-0020639 discloses a composition containing *Salvia miltiorrhiza* Bunge for treatment of prostate cancer, and Korean Patent Publication No. 2001-0076810 discloses a method for preparing a composition for treatment of prostatic diseases and hemorrhoids and a composition prepared by the method, wherein a pill is prepared by mixing *Polygonum cuspidatum, Portulaca oleracea, Alpinia oxyphylla, Plantago asiatica, Taraxacum platycarpum, Lonicera japonica, Gentiana scabra, Rheum palmatum, Gallus gallus, Caesalpinia sappan, Chaenomeles sinensis, Melia azedarach, Trogopterus xanthipes, Salvia miltiorrhiza* Bunge, *Commiphora myrrha, Psoralea corylifolia, Clematis manshurica, Boswellia carterii, Dryobalanops aromatica, Corydalis ternata, Chelidonium majus, Foeniculum vulgare, Scirpus flaviatilis, Curcuma zedoaria, Cnidium monnieri, Cyperus rotundus, Lindera strychnifolium, Pheretima aspergillum,* and *Whitmania pigra* each 2-5 wt%, but does not disclose the composition containing *Salvia miltiorrhiza* Bunge as an active ingredient for prevention and treatment of benign prostatic hyperplasia.

In addition, Korea Patent Registration No. 0259037 discloses an externally-applied liquid preparation for promoting hair growth, wherein the externally-applied liquid preparation is prepared by using extracts obtained by immersing powders of *Pinellia ternate, Syzygium aromaticum, Rubus coreanus, Zanthoxylum bungeanum, Vitex rotundifolia, Salvia miltiorrhiza* Bunge and *Thuja orientalis* in oil and ethanol, respectively, followed by aging for a fixed period of time, but these features are different from those of the present invention.

### [Prior Art Documents]

### [Patent Documents]

Korean Patent Publication No. 2012-0020639, Composition containing *Salvia* miltiorrhiza Bunge for treatment of prostate cancer, 8 March 2012.

Korean Patent Publication No. 2001-0076810, method for preparing composition for treatment of prostatic diseases and hemorrhoids and composition prepared thereby, 16 August 2001.

Korea Patent Registration No. 0259037, externally-applied liquid preparation for promoting hair growth, 16 March 2000.

### [Non-Patent Documents]

Berry, S.J.; Coffey, D.S.; Walsh, P.C.; Ewing, L.L. The development of human benign prostatic hyperplasia with age. J. Urol. 1984, 132, 474-479.

Sarma, A.V.; Wei, J.T. Clinical practice. Benign prostatic hyperplasia and lower urinary tract symptoms. N. Engl. J. Med. 2012, 367, 248-257.

Kim, S.K.; Seok, H.; Park, H.J.; Jeon, H.S.; Kang, S.W.; Lee, B.C.; Yi, J.; Song, S.Y.; Lee, S.H.; Kim, Y.O.; et al. Inhibitory effect of curcumin on testosterone induced benign prostatic hyperplasia rat model. BMC Complement. Altern. Med. 2015, 15, 380-386.

Choi, H.M.; Jung, Y.; Park, J.B.; Kim, H.L.; Youn, D.H.; Kang, J.W.; Jeong, M.Y.; Lee, J.H.; Yang, W.M.; Lee, S.G.; et al. Cinnamomi cortex (Cinnamomum verum) suppresses testosterone-induced benign prostatic hyperplasia by regulating 5α-reductase. Sci. Rep. 2016, 6, 31906-31917.

Chen, Y.; Robles, A.I.; Martinez, L.A.; Liu, F.; Gimenez-Conti, I.B.; Conti, C.J. Expression of G1 cyclins, cyclin-dependent kinases, and cyclin-dependent kinase inhibitors in androgen-induced prostate proliferation in castrated rats. Cell Growth Differ. 1996, 7, 1571-1578.

Wang, W.; Bergh, A.; Damber, J.E. Chronic inflammation in benign prostate hyperplasia is associated with focal upregulation of cyclooxygenase-2, Bcl-2, and cell proliferation in the glandular epithelium. Prostate 2004, 61, 60-72.

Cardillo, M.; Berchem, G.; Tarkington, M.A.; Krajewski, S.; Krajewski, M.; Reed, J.C.; Tehan, T.; Ortega, L.; Lage, J.; Gelmann, E.P. Resistance to apoptosis and up regulation of Bcl-2 in benign prostatic hyperplasia after androgen deprivation. J. Urol. 1997, 158, 212-216.

Dennis A. Holt; Mark A. Levy; Hye Ja Oh; Jill M. Erb; Julie I.; Heaslip; Martin Brandt; Hsuan Yin Lan-Hargest; Brian W. Metcalf, Inhibition of steroid 5.alpha.-reductase by unsaturated 3-carboxy steroids, J. Med. Chem. 1990, 333, 943-950.

Park, E.S.; Lee, M.Y.; Jeon, W.W.; Seo, C.S.; You, S.S.; Shin, H.K. Paljung-San, a traditional herbal medicine, attenuates benign prostatic hyperplasia in vitro and in vivo. J. Ethnopharmacol. 2018, 218, 109-115.

Marks, L.S.; Partin, A.W.; Epstein, J.I.; Tyler, V.E.; Simon, I.; Macairan, M.L.; Chan, T.L.; Dorey, F.J.; Garris, J.B.; Veltri, R.W.; et al. Effects of a saw palmetto herbal blend in men with symptomatic benign prostatic hyperplasia. J. Urol. 2000, 163, 1451-1456.

SHAN GAO, SHIQIN LI, QIN LI, FUYONG ZHANG, MENGQI SUN, ZILIN WAN and SHURONG WANG, Protective effects of salvianolic acid B against hydrogen peroxide-induced apoptosis of human umbilical vein endothelial cells and underlying mechanisms, INTERNATIONAL JOURNAL OF MOlecular medicine 44: 457-468, 2019

### Detailed Description of the Invention

### Technical Problem

An aspect of the present invention is to provide a composition containing a *Salvia miltiorrhiza* Bunge extract as an active ingredient for treatment or prevention of benign prostatic hyperplasia.

### Technical Solution

In accordance with an aspect of the present invention, there is provided a composition containing a *Salvia miltiorrhiza* Bunge extract as an active ingredient for treatment or prevention of benign prostatic hyperplasia or alopecia.

The *Salvia miltiorrhiza* Bunge extract may be a distilled water or ethanol extract, or other solvents may be used. The extraction temperature, the extraction time, the amount of the solvent, the treatment manner of residual components may be differently designed depending on the kind of extraction solvent. Various solvents may be used as an extraction solvent, and examples of extractable solvents include water, ethanol, methanol, a fatty oil, glycerin, a horse oil, ethyl acetate, acetone, butanol, isopropanol, methylene chloride, and the like.

The *Salvia miltiorrhiza* Bunge extract may be especially an extract extracted with an alcohol having one to six carbon atoms or a mixed solvent thereof. The alcohol may be ethanol, or may be 40-99% ethanol.

The *Salvia miltiorrhiza* Bunge extract may be a 40-99% ethanol extract.

The *Salvia miltiorrhiza* Bunge extract may be concentrated. The extract may be heated at 40-80°C by microbar drying to thereby remove moisture therefrom. Alternately, the extract may be concentrated and dried by low-temperature vacuum drying. Low-temperature vacuum drying is a drying method conducted while the pressure inside a dryer is maintained in a vacuum state and the temperature is adjusted to 5-15°C, and does not cause the denaturation of extracted components and the loss of taste and aroma. Spray drying, cold-air drying, hot-air drying, freeze drying, far-infrared drying, shade drying, drying under reduced pressure, or the like may be used according to the design conditions.

Alternatively, *Salvia miltiorrhiza* Bunge may be processed using cryogenic micro grinding technology (CMGT). More specifically, *Salvia miltiorrhiza* Bunge roots may be frozen at a cryogenic temperature of -196 to -80°C for a short time and ground to a very fine powder, thereby preparing a powder having improved properties.

The *Salvia miltiorrhiza* Bunge concentrate may be dried and prepared into various formulations. The extract may be concentrated and dried by spray drying, and then prepared into various formulations. For example, the extract may be prepared into a powder. Low-temperature vacuum drying is a drying method conducted while the pressure inside a dryer is maintained in a vacuum state and the temperature is adjusted to 5-15°C, and does not cause the denaturation of extracted components and the loss of taste and aroma. Cold-air drying, hot-air drying, freeze drying, far-infrared drying, shade drying, drying under reduced pressure, or the like may be used according to the design conditions. Preferably, *S. miltiorrhiza* may be eluted with 50 1 of ethanol for 24 hours, and then concentrated under reduced pressure. To this, 1500 ml of water is added, and the same amount of n-hexane, dichloromethane (CH₂Cl₂), and ethyl acetate (EtOAc) are added, followed by repetitive extraction twice, thereby preparing a gel-state red extract of *S. miltiorrhiza.*

The extract may be a distilled water or ethanol lysate of cryogenically frozen fine powder of *S. miltiorrhiza.* However, without limitation thereto, S. *miltiorrhiza* may be subjected to extraction by a usable known extraction method, such as hot-water extraction, distillation extraction, ethanol extraction, ultrasonic ethanol extraction, or ultrasonic water extraction. The *S. miltiorrhiza* extract may be contained at an amount of 0.01-700 µg/ml.

Through the above methods, the content of tanshinones, which are effective components of S. *miltiorrhiza,* can be increased. Tanshinones may be contained in 2-70% of the total weight of the *S. miltiorrhiza* Bunge extract.

The *S. miltiorrhiza* Bunge extract may be subjected to fractional extraction with an organic solvent including methylene chloride, and particular components may be concentrated through a column.

The tanshinones may be contained in 2-70% of the total weight of the *Salvia miltiorrhiza* Bunge extract.

The tanshinones may include at least one of a group consisting of 1,6-dimethylphenanthro[1,2-b]furan-10,11-dione or 1,6-dimethylnaphtho[1,2-g]benzofuran-10,11-dione) (tanshinone1), 1,6,6-trimethyl-6,7,8,9-tetrahydrophenanthro[1,2-b] furan-10, 11-dione (tanshinone2a), (R)-1,2,6,7,8,9-hexahydro-1,6,6-trimethyl-phenanthro(1,2-b)furan-10, 11-dione (cryptotanshinone), and (1R)-1,6-dimethyl-1,2-dihydronaphtho[1,2-g][1]benzofuran-10,11-dione dihydrotanshinone-I (15,16-dihydrotanshinone), or may be tanshinones excluding the above four types of tanshinones.

The present invention provides a composition for treatment or prevention of benign prostatic hyperplasia or alopecia, the composition containing as an active ingredient at least one of the group consisting of tanshinone1, tanshinone2a, cryptotanshinone, dihydrotanshinone, and tanshinoic acid.

Examples of the main tanshinones that have been known until now include tanshinone1, tanshinone2a, cryptotanshinone, or dihydrotanshinone, and other tanshinones having different end functional groups, including tanshinoic acid, are included. The effective components of *Salvia miltiorrhiza* Bunge may be tanshinone1, tanshinone2a, cryptotanshinone, or dihydrotanshinone, preferably tanshinone2a, dihydrotanshinone, or other tanshinones including tanshinoic acid, and more preferably dihydrotanshinone or other tanshinones.

The present invention provides a pharmaceutical composition containing a *Salvia miltiorrhiza* Bunge extract as an active ingredient for treatment or prevention of benign prostatic hyperplasia. The pharmaceutical composition of the present invention may contain a *Salvia miltiorrhiza* Bunge extract or effective components of *Salvia miltiorrhiza* Bunge, including tanshinone1, tanshinone2a, cryptotanshinone, dihydrotanshinone, and/or other tanshinones, such as tanshinoic acid.

The pharmaceutical composition may contain a carrier or an excipient, each of which may be added in preferably 0.001-90 wt%, more preferably 0.001-50 wt%, and most preferably 0.001-30 wt%, relative to the total weight of the pharmaceutical composition.

The suitable dose of the pharmaceutical composition may vary depending on various factors, such as method of preparation, manner of administration, and patient's age, body weight, sex, morbidity, diet, administration time, administration route, excretion rate, and response sensitivity. The pharmaceutical composition of the present invention contains the tanshinone derivatives as active ingredients. The pharmaceutical composition can be orally or parenterally administered upon clinical administration, and may be used in the form of a general medicinal preparation. That is, the composition of the present invention may be administered in several oral and parenteral formulations upon actual clinical administration, and the composition of the present invention, when made into a preparation, may be prepared using a diluent or an excipient, such as a filler, an extender, a binder, a wetting agent, a disintegrant, and a surfactant, that are usually used. Examples of solid preparations for oral administration include a tablet, a pill, a powder, granules, a capsule, and the like, and such solid preparations are prepared by mixing the tanshinone derivatives with at least one vehicle, such as starch, calcium carbonate, sucrose, lactose, and gelatin. Alternatively, in addition to the simple vehicle, lubricants, such as magnesium stearate and talc, may be used. Examples of liquid preparations for oral administration may include a suspension, a liquid preparation for internal use, an emulsion, a syrup, and the like. In addition to simple diluents that are frequently used, such as water and liquid paraffin, several excipients, for example, a wetting agent, a sweetener, an aroma, a preservative, and the like may be contained in the liquid preparations. Examples of a preparation for parenteral administration include a sterile aqueous solution, a non-aqueous solvent, a suspension, an emulsion, a lyophilizer, and a suppository. Examples of the non-aqueous solvent and the suspension solvent may include propylene glycol, polyethylene glycol, a vegetable oil like olive oil, an injectable ester like ethylolate, and others. Examples of a substrate for the suppository may include Witepsol, macrogol, Tween 61, cocoa butter, laurin butter, glycerol, gelatin, or the like.

The dosage units can contain, for example, 1, 2, 3 or 4 times the individual dose or 1/2, 1/3, or 1/4 times the individual dose. An individual dose preferably contains the amount of active compound which is given in one administration and which usually corresponds to a whole, a half or a third, or a quarter of a daily dose. The effective dosage of the tanshinone derivatives is dose-dependent, but preferably 0.1-1,000 mg/kg and more preferably 0.4-500 mg/kg, and the administration frequency is 1-6 times a day. Therefore, the tanshinone derivatives may be administered in a range of 0.1-6,000 mg/day per 1 kg of adult body weight.

The pharmaceutical composition may be administered to mammals, such as rats, domestic animals, and humans, via various routes. All moods of administration may be contemplated, for example, administration may be carried out orally, rectally, or by intravenous, intramuscular, subcutaneous, intrauterine, epidural or intracerebroventricular injection.

The *Salvia miltiorrhiza* Bunge extract can reduce the expression of at least one protein of the group consisting of 5α-reductase type 2 (5AR2), prostate-specific antigen (PSA), steroid receptor co-activator-1 (SRC-1), and androgen receptor (AR). The proteins are androgen-related proteins, and show an increase tendency in benign prostatic hyperplasia or alopecia.

The present invention provides a composition for treatment or prevention of benign prostatic hyperplasia or alopecia, wherein the *Salvia miltiorrhiza* Bunge extract is prepared by a method for preparing a *Salvia miltiorrhiza* Bunge extract, the method including:
(1) crushing *Salvia miltiorrhiza* Bunge with impurities removed therefrom;
(2) immersing the *Salvia miltiorrhiza* Bunge crushed in step (1) in 40-99% ethanol (EtOH) at 50-80°C, followed by standing for 2-24 hours;
(3) separating the ethanol after the extraction from *Salvia miltiorrhiza* Bunge in step (2), and adding new ethanol to residues to immerse the residues in a 40-99% ethanol (EtOH) at 50-80°C, followed by standing for 2-12 hours;
(4) separating the ethanol after the extraction from *Salvia miltiorrhiza* Bunge in step (3), and adding new ethanol to residues to immerse the residues in a 40-99% ethanol (EtOH) at 50-80°C, followed by standing for 2-5 hours;
(5) collecting the ethanol separated in each of steps (2) to (4), followed by evaporation and drying at 45-75°C; and
(6) grinding a solid extract obtained from the evaporation and drying in step (5), followed by filtration at 80 mesh or greater, to obtain the *Salvia miltiorrhiza* Bunge extract.

In addition, the present inveniton provides a composition for treatment or prevention of benign prostatic hyperplasia or alopecia, wherein in step (1) of crushing *Salvia miltiorrhiza* Bunge with impurities removed therefrom, *Salvia miltiorrhiza* Bunge is ground by cryogenic micro grinding technology (CMGT) in which *Salvia miltiorrhiza* Bunge is frozen at a cryogenic temperature of -196 to -80°C for a short time and ground to 5 mm or less.

In accordance with another aspect of the present invention, there is provided a health functional food containing a *Salvia miltiorrhiza* Bunge extract as an active ingredient for prevention or alleviation of benign prostatic hyperplasia or alopecia.

The health functional food provides a health functional food containing a *Salvia miltiorrhiza* Bunge extract, effective components of *Salvia miltiorrhiza* Bunge, including tanshinone1, tanshinone2a, cryptotanshinone, dihydrotanshinone, or other tanshinones such as tanshinoic acid, and a sitologically acceptable food supplement additive.

In the health functional food, the *Salvia miltiorrhiza* Bunge extract, and effective components of *Salvia miltiorrhiza* Bunge, including tanshinone1, tanshinone2a, cryptotanshinone, dihydrotanshinone, and/or other tanshinones such as tanshinoic acid, may be added, relative to the total weight of the health functional food, in preferably 0.001-50 wt%, more preferably 0.001-30 wt%, and most preferably 0.001-10 wt%.

The *Salvia miltiorrhiza* Bunge extract of the present invention may be added, relative to the total weight of the health functional food, in preferably 0.001-50 wt%, more preferably 0.001-30 wt%, and most preferably 0.001-10 wt%.

The health functional food encompasses the form of a tablet, a capsule, a pill, or a liquid preparation, and examples of a food to which the extract of the present invention can be added include meat, sausage, bread, chocolate, candies, snacks, confectionery, pizza, ramen, other noodles, gums, powdered milk, mixed grain powder, raw food, lactic acid fermented milk, dairy products including ice cream, various soups, beverages, teas, drinks, alcoholic beverages, and vitamin complexes. Specifically, examples of the health food containing a *Salvia miltiorrhiza* Bunge extract may include health foods and favorite foods, such as juice, tea, jelly, and juice, which contain a *Salvia miltiorrhiza* Bunge extract as an active ingredient, and folk remedies for edema, nephritis, urethritis, and the like may be included.

The composition containing a *Salvia miltiorrhiza* Bunge extract as an active ingredient for treatment or prevention of benign prostatic hyperplasia may be prepared into a cosmetic composition. When such a composition is used as a cosmetic composition, a *Salvia miltiorrhiza* Bunge extract may be used per se, or other cosmetic components may be used together, and these may be appropriately used according to a typical method. The amounts of the active ingredient mixed may be suitably determined according to the purpose of use thereof, and typically, upon the manufacture of a cosmetic product using a *Salvia miltiorrhiza* Bunge extract, the active ingredient may be added, relative to the total weight of raw materials, at the amount of 0.0001-10 wt%, and preferably 0.1-5 wt%. For cosmetic products, a cosmetic composition can have any one formulation selected from the group consisting of an ointment for external skin application, a creme, a softening cosmetic water, a nutritional cosmetic water, a pack, an essence, a hair toner, a shampoo, a rinse, a hair conditioner, a hair treatment, a gel, a skin lotion, a skin softener, a skin toner, an astringent, a lotion, a milk lotion, a moisture lotion, a nutritional lotion, a massage creme, a nutritional creme, a moisture creme, a hand creme, a foundation, a color cosmetic product, a nutritional essence, a sunscreen, a soap, a cleansing foam, a cleansing lotion, a cleansing creme, a body lotion, and a body cleanser, but is not limited thereto.

In cases where the formulation of the present invention is a surfactant-containing cleansing, aliphatic alcohol sulfate, aliphatic alcohol ether sulfate, sulfosuccinate monoester, isethionate, imidazolium derivatives, methyl taurate, sarcosinate, fatty acid amide ether sulfate, alkyl amido betaine, aliphatic alcohol, fatty acid glyceride, fatty acid diethanolamide, vegetable oils, lanoline derivatives, or ethoxylated glycerol fatty acid ester may be used as a carrier ingredient.

The formulation of the present invention may additionally contain excipients including fluorescent substances, fungicides, hydrotropic substances, moisturizers, aromas, aroma carriers, proteins, solubilizing agents, sugar derivatives, sunscreens, vitamins, plant extracts, and the like.

### Advantageous Effects

As described above, according to the present invention, the composition containing a *Salvia miltiorrhiza* Bunge extract as an active ingredient for treatment or prevention of benign prostatic hyperplasia or alopecia may provide a safe and potent composition for treatment and prevention of benign prostatic hyperplasia, which can substitute for conventional medicines having many side effects.

### Brief Description of the Drawings

FIG. 1 shows the Western blotting results of verifying expression changes of 5α-reductase type 2 (5AR2), androgen receptor (AR), steroid receptor coactivator-1 (SRC-1), and prostate specific antigen (PSA) by *Salvia miltiorrhiza* Bunge extract S containing salvianolic acid as a main ingredient (about 10-20%), *Salvia miltiorrhiza* Bunge extract T containing tanshinones as main ingredients (about 22%), and as effective components thereof, tanshinone1 (Tan1), tanshinones 2a (Tan2a), cryptotanshinone (CryT), and dihydrotanshinone (DihT) in DHT-induced LNCaP cells.
FIG. 2 shows graphs depicting the quantification of each protein in FIG. 1.
FIG. 3 shows the Western blotting results of depicting effects on the expression of androgen receptor (AR) and prostate-specific antigen (PSA) when DHT-treated LNCaP cells were treated with different concentrations of *Salvia miltiorrhiza* Bunge extracts T95, T70, and T40.
FIG. 4 shows graphs depicting the quantification of androgen receptor (AR) (A) and prostate-specific antigen (PSA) (B) each in FIG. 3.
FIG. 5 shows a graph depicting body weight changes of rats by treatment with *Salvia miltiorrhiza* Bunge extracts T70 to T10 in benign prostatic hyperplasia animal models.
FIG. 6 shows photographs depicting prostate size changes of rats by the treatment with *Salvia miltiorrhiza* Bunge extracts T70 to T10 in benign prostatic hyperplasia animal models.
FIG. 7 shows a graph depicting prostate weight changes of rats by treatment with *Salvia miltiorrhiza* Bunge extracts T70 to T10 in benign prostatic hyperplasia animal models.
FIG. 8 shows a graph depicting prostate index (prostate weight compared with body weight) changes of rats by treatment with *Salvia miltiorrhiza* Bunge extracts T70 to T10 in benign prostatic hyperplasia animal models.
FIG. 9 shows the Western blotting results depicting the effects of *Salvia miltiorrhiza* Bunge extract T40 on the expression of 5α-reductase (5AR2), androgen receptor (AR), and prostate-specific antigen (PSA), which are benign prostate hyperplasia-related proteins of rats, in benign prostate hyperplasia animal models.
FIG. 10 is a schematic diagram showing the signaling pathway of testosterone and pharmacological mechanism of *Salvia miltiorrhiza* Bunge extracts in BPH.

### Mode for Carrying Out the Invention

Benign prostatic hyperplasia (BPH) is a common chronic disease of the urinary system among elderly men. Especially, the metabolic imbalance of androgen in elderly men is one of the leading causes of BPH. Dihydrotestosterone (DHT) by 5α reductase type 2 (5AR2), binding with androgen receptor (AR), affect prostate proliferation and growth. In BPH, levels of androgen signaling-related protein expression are reported to be high, and androgen signaling is associated with the overexpression of prostate-specific antigen (PSA). Another symptom associated with the regulation of dihydrotestosterone (DHT) is alopecia.

The present inventors, while researching signaling pathways of benign prostatic hyperplasia (BPH) and alopecia, have verified that a *Salvia miltiorrhiza* Bunge extract and effective components thereof are involved in corresponding pathways and thus have excellent effect on the treatment and prevention of benign prostatic hyperplasia (BPH) and alopecia, and have completed the present invention.

Hereinafter, preferable examples of the present invention will be described in detail. However, the present invention is not limited to the examples described herein and can be embodied in many different forms. Rather, these examples are provided so that the present disclosure will be thorough and complete and will fully convey the scope of the disclosure to those skilled in the art.

### <Example 1: Component analysis of Salvia miltiorrhiza Bunge extracts>

### 1.1. Preparation of Salvia miltiorrhiza Bunge extract S

The crushed *Salvia miltiorrhiza* Bunge roots were extracted in hot water at 50-100°C. After extraction with the hot water for 2-24 hours, debris were removed by filtration, and the extract was collected, heated, and dried by evaporation. The solids after evaporation were washed with hot water of 100°C as necessary, and then dried in the microwave at 55°C for 25-30 minutes, ground, and then filtered with 60 mesh or greater. A main component of the *Salvia miltiorrhiza* Bunge extract S extracted from *Salvia miltiorrhiza* Bunge through the step is salvianolic acid, which was showed to account for about 10-20% of the extract.

For the increase in extraction efficiency of *Salvia miltiorrhiza* Bunge, the *Salvia miltiorrhiza* Bunge roots may be processed using cryogenic micro grinding technology (CMGT). Cryogenic micro grinding technology (CMGT) is the processing technology in which natural products or food materials are rapidly frozen and ground in a cryogenic temperature condition of -196°C to minimize changes in intrinsic properties, taste, nutrition, aroma, and the like. CMGT enables the processing of materials that are difficult to grind at room temperature or have a high fat content, materials with a high content of moisture, and the like, and the improvement of dissolving power and dispersing power without damaging original properties. CMGT is also an impact-type grinding method in which raw materials are cooled at a cryogenic temperature to be effective against brittle fracture (remarkably brittle by impact). CMGT makes particles become circular due to the grinding by friction between the particles, and results in a small denaturation and a low grinding heat compared with other grinding types under the same particle size, allowing continuous grinding of materials have a large fat content, and therefore, CGMT can be easily applied to food materials. Large-sized raw materials were ground to 5 mm or smaller in a coarse grinder (Duksan, Korea) before use, and raw materials having such a size that a screw manufactured by Hosokawa (Linrex Mill LX-1, Japan) and a screw manufactured by Duksan can be used were ground as they are and then used.

The *Salvia miltiorrhiza* Bunge roots were processed using cryogenic micro grinding technology (CMGT) to manufacture *Salvia miltiorrhiza* Bunge CMGT. More specifically, the *Salvia miltiorrhiza* Bunge roots were frozen at a cryogenic temperature of -110 to -90°C for a short time, ground at 4500-5500 rpm, and then used in the manufacture of *Salvia miltiorrhiza* Bunge extract S, and *Salvia miltiorrhiza* Bunge extract T below.

### 1.2. Preparation of Salvia miltiorrhiza Bunge extract T

*Salvia miltiorrhiza* Bunge extract T was extracted from *Salvia miltiorrhiza* Bunge roots at 50-80°C using ethanol (EtOH) (*Salvia miltiorrhiza* Bunge extract T). To investigate effects according to the concentration of the extraction ethanol, *Salvia miltiorrhiza* Bunge extracts T10 (EtOH 10%), T40 (EtOH 40%), T70 (EtOH 70%), T95 (EtOH 95%), and T99 (EtOH 99%) were obtained using different concentrations of ethanol. Extraction was conducted using each extract for 2-24 hours, and as necessary, extraction was again conducted using new ethanol for 2-12 hours, and as necessary, extraction was further conducted using new ethanol for 2-5 hours, followed by centrifugation, to obtain a supernatant. Thereafter, the obtained supernatant was collected, evaporated at 45-75°C, dried, ground, and then filtered with 80 mesh or greater, thereby preparing each extract.

### 1.3. Component analysis of Salvia miltiorrhiza Bunge extracts

*Salvia miltiorrhiza* Bunge extract S and *Salvia miltiorrhiza* Bunge extract T extracted in Examples 1.1 and 1.2 were measured for components and contents. Especially, in order to set four types of main tanshinone components contained in *Salvia miltiorrhiza* Bunge, it was investigated through HPLC assay whether tanshinone1, tanshinone2a, cryptotanshinone, dihydrotanshinone, salvianolic acid B, and the like, which were sold as standard products, among various compounds revealed by existing researchers, were present in the *Salvia miltiorrhiza* Bunge extracts. HPLC Conditions were as follows: Column; cadeza cd-C18 3.0 *µ*m, 3.0 mm × 250 mm, Detector; DAD (254 nm), Temp.; 40°C, Injection Volume; 10 µL, Flow rate; 0.2 mL/min, Mobile phase; 80% MeOH.

*Salvia miltiorrhiza* Bunge extract contain various tanshinones in addition to main tanshinone components. Other tanshinone standard products are difficult to purchase. In general, tanshinones have a common framework in terms of molecular structures and have constant molecular weights (molecular weight of around 300 kDa) and similar physicochemical properties. On the basis of these facts, Tanshinone2a was set as an index component, and then the concentration-specific absorbance standard curve was established. The absorbance was measured in the 270 nm wavelength band using a spectrophotometer and the total tanshinones were quantified by a regression equation, and tabulated in Table 1 below.

**TABLE 1**

| Compound | *Salvia miltiorrhiza* Bunge extract S | *Salvia miltiorrhiza* Bunge extract T | | | |
|---|---|---|---|---|---|
| | | T10 | T40 | T70 | T95 |
| Total Tanshinones | ≪ 0.1% | 1.72% | 10.79% | 19.34% | 22.22% |
| 15,16-Dihydrotanshinone | ≪ 0.1% | ≪ 0.1% | 0.64% | 1.31% | 1.75% |
| Tanshinonel | ≪ 0.1% | 0.20% | 1.22% | 1.84% | 2.03% |
| Tanshinone2a | ≪ 0.1% | 0.28% | 1.76% | 2.81% | 3.11% |
| Cryptotanshinone | ≪ 0.1% | 0.50% | 2.62% | 5.16% | 5.88% |
| Other Tanshinones | ≪ 0.1% | 0.74% | 4.55% | 8.22% | 9.45% |
| Salvianolic acid B | 15.14% | 12.98% | 2.54% | ≪0.1% | ≪0.1% |

As shown in Table 1 above, tanshinones were little detected and 15.4% of salvianolic acid B was detected in *Salvia miltiorrhiza* Bunge extract S, which was a hot-water extract. Total tanshinones were extracted to have 1.72% in 10% ethanol and 10.79% in 40% ethanol. It was verified that when the ethanol concentration was higher, the extraction efficiency of tanshinones was also higher. Total tanshinones were contained in 22.22% in the extract under a solvent condition of 95% ethanol. On the contrary, the content of salvianolic acid B rapidly decreased as the concentration of ethanol increased.

Among the tanshinones, cryptotanshinone was most detected, followed by tanshinone2a, tanshinone1, and 15,16-dihydrotanshinone. Other tanshinones except the main tanshinones also increased as the concentration of ethanol increased. *Salvia miltiorrhiza* Bunge extract T99 also showed similar results to *Salvia miltiorrhiza* Bunge extract T95, but *Salvia miltiorrhiza* Bunge extract T99 was highly unstable during heating in the extraction procedure, resulting in low efficiency in mass production. Thus, *Salvia miltiorrhiza* Bunge extract T95 was used as a representative.

### <Example 3: Verification of effect of Salvia miltiorrhiza Bunge extracts on androgenic pathway-related protein level in DHT-treated LNCaP cells>

### 3.1. Cell culture

Animal cells were cultured to investigate the effect of the *Salvia miltiorrhiza* Bunge extract of the present invention on benign prostatic hyperplasia or alopecia. Human prostate adenocarcinoma cell line LNCaP cells sensitive to androgens were used as animal cells, and purchased from the Korean Cell Line Bank (Seoul, Korea, KCLB #:21740). The cells were cultured in Roswell Park Memorial Institute (RPMI) medium supplemented with 100 mg/ml penicillin/streptomycin and 10% fetal bovine serum (FBS), and maintained in a CO₂ incubator at 37°C.

### 3.2. Materials

Goat anti-rabbit immunoglobulin G (IgG, 7074) and anti-mouse IgG (7076) were purchased from Cell Signaling (Danvers, MA). Antibodies against AR (SC-816), SRC1 (SC-32789), PSA (SC-7638), and β-actin (SC-1616) were purchased from Santa Cruz Biotechnology (Dallas, TX, USA), and antibodies against 5AR2 (ab124877) were purchased from Abcam Inc. (Cambridge, MA, USA). Roswell Park Memorial Institute (RPMI) medium, fetal bovine serum (FBS), and penicillin/streptomycin were purchased from Gibco (Big Cabin, OK, USA). Reagents including finasteride (Fi, ≥97% pure) and DHT (≥ 99% pure) were purchased from Sigma-Aldrich Inc. (St. Louis, MO, USA).

### 3.3. Effect of Salvia miltiorrhiza Bunge extract on DHT-treated LNCaP cells

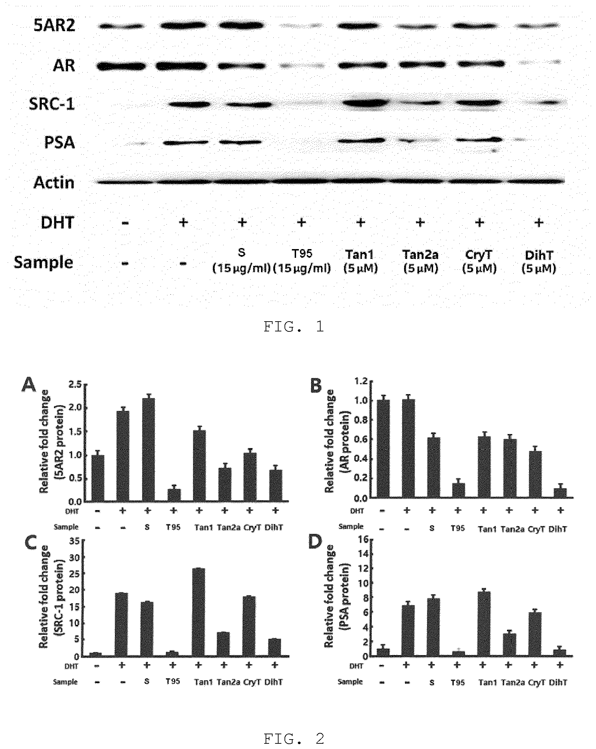

The LNCaP cells were seeded onto 6-well plates (1 × 10⁶ cells/well) in 2 mL of RPMI medium supplemented with 10% FBS, 100 U/mL penicillin, and 100 mg/ml streptomycin. One day later, the cells were treated with DHT (10 nmol), *Salvia miltiorrhiza* Bunge extract S and *Salvia miltiorrhiza* Bunge extract T95 at 15 µg/ml, tanshinone1, tanshinone2a, cryptotanshinone and 15,16-dihydrotanshinone at 5 µM. The harvested LNCaP cells were lysed using a cold radioimmunoprecipitation assay (RIPA) buffer containing a protease inhibitor cocktail. The lysed cells were centrifuged at 13,000 RPM for 20 minutes at 4°C, and the protein concentration was determined using BCA assay. The cell lysates (30 µg protein/sample) were separated by 10% sodium dodecyl sulfate-polyacrylamide gel electrophoresis (SDS-PAGE) at 120% for 90 minutes and transferred onto nitrocellulose membranes. The membranes were blocked with 5% skim milk at room temperature for 1 hour. Immediately afterward, primary antibodies (diluted to 1:2000), such as AR, 5AR2, PSA, and SRC-1 were reacted overnight to the membrane. After washed, the membranes were incubated with goat anti-rabbit IgG and anti-mouse IgG horseradish peroxidase (HRP)-conjugated secondary antibody (diluted to 1:10000) for 1 hour at room temperature. Immunodetection was performed using an enhanced chemiluminescence (ECL) detection reagent, and the results are shown in FIG. 1. Thereafter, the membranes were photographed using Davinch-Chemi Imaging System (Davinch-K., Seoul). The chemiluminescent intensities of protein signals were quantified using ImageJ 1.47v software, and the results are shown in FIG. 2. The data obtained by SPSS version 11.5 for Windows (SPSS Inc., Chicago, IL, USA) were expressed as the means ± standard errors of the means (SEM). The means of two continuous normally distributed variables were compared by the Student's t-test for independent samples. Dunnett's multiple range tests were used to compare the means of two and three or more groups of variables that did not follow normal distribution. *p*<0.05 and *p*< 0.01 were considered as the criteria for statistical significance.

As shown in FIGS. 1 and 2, the expression levels of androgen-related proteins, excluding androgen receptor (AR), were increased by DHT in LNCaP cells. The treatment with *Salvia miltiorrhiza* Bunge extract S somewhat reduced the expression of SRC-1 increased by DHT, and reduced the expression level of androgen receptor (AR) by 40%. However, 5α reductase type 2 (5AR2) and prostate-specific antigen (PSA) significantly increased in benign prostatic hyperplasia showed an increased expression tendency. However, *Salvia miltiorrhiza* Bunge extract T significantly reduced the protein expression of all of 5α reductase type 2 (5AR2), prostate-specific antigen (PSA), steroid receptor coactivator-1 (SRC-1), and androgen receptor (AR). Especially, *Salvia miltiorrhiza* Bunge extract T inhibited the expression of 5α-reductase type 2 (5AR2), prostate-specific antigen (PSA), and androgen receptor (AR) to lower levels than a control group.

When DHT-induced LNCaP cells were treated with 5 µM each of tanshinone1, tanshinone2a, cryptotanshinone, and 15,16-dihydrotanshinone, which were confirmed as main tanshinones in *Salvia miltiorrhiza* Bunge, 15,16-dihydrotanshinone showed a most inhibitory effect on androgen-related proteins increased by DHT. Especially, 15,16-dihydrotanshinone of the same concentration inhibited the expression of prostate-specific antigen (PSA) and androgen receptor (AR) at least 5 times than tanshinone2a. This indicates that a composition effective for the treatment and prevention of a disease mediated by androgen-related proteins, such as benign prostatic hyperplasia or alopecia can be provided. 15,16-dihydrotanshinone also inhibited the expression of 5α-reductase type 2 (5AR2) and steroid receptor coactivator-1 (SRC-1) more effectively than tanshinone2a. Meanwhile, the treatment with 15 µg/ml *Salvia miltiorrhiza* Bunge extract T inhibited the expression of androgen-related proteins more effectively than 15,16-dihydrotanshinone. In order to investigate whether these results are due to a difference in concentration, the contents of individual tanshinones in 15 µg/ml *Salvia miltiorrhiza* Bunge extract T were converted on the basis of the results of Table 1, and tabulated in Table 2.

**TABLE 2**

| Compound | *Salvia miltiorrhiza* Bunge extract T95 | |
|---|---|---|
| Total Tanshinones | 15 *µ*g/ml | 3.33*µ*g/ml |
| 15,16-Dihydrotanshinone | | 0.26*µ*g/ml |
| Tanshinonel | | 0.30*µ*g/ml |
| Tanshinone2a | | 0.47*µ*g/ml |
| Cryptotanshinone | | 0.89*µ*g/ml |
| Other Tanshinones | | 1.42*µ*g/ml |

As shown in Table 2, 3.33 µg/ml tanshinones were contained in *Salvia miltiorrhiza* Bunge extract T, and the content of 15,16-dihydrotanshinone showing a most remarkable inhibitory effect on androgen-related proteins was only 0.26 µg/ml in the tanshinones, but showed a more remarkable inhibitory effect than other main tanshinones. These results indicate that a mixture of several types of tanshinones contained in *Salvia miltiorrhiza* Bunge extract T showed a synergistic effect or a compound having an excellent inhibitory effect on androgen-related proteins has a possibility of being included in other tanshinones excluding four types of main tanshinones.

### 3.4. Effects of different concentrations of Salvia miltiorrhiza Bunge extract T on DHT-treated LNCaP cells

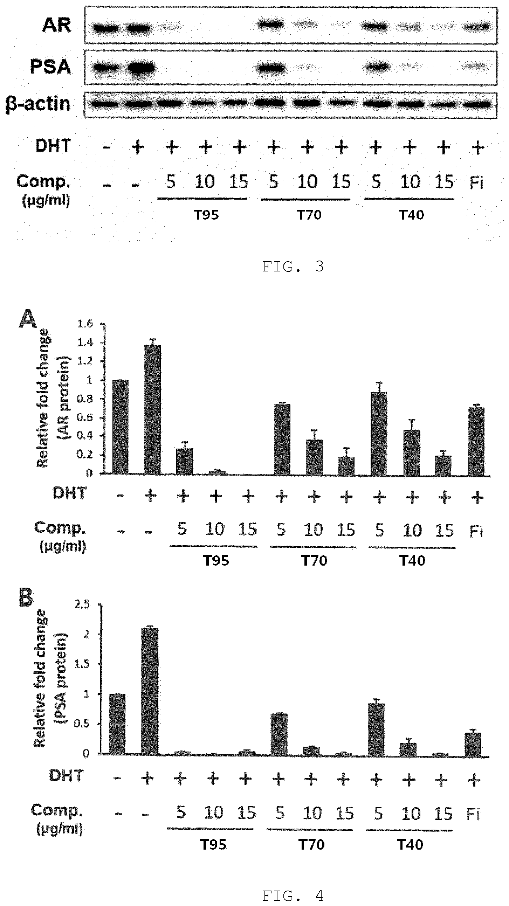

In order to investigate inhibitory effects of *Salvia miltiorrhiza* Bunge extract T according to ethanol concentration on androgen-related proteins, DHT-treated LNCaP cells were treated with different concentrations of *Salvia miltiorrhiza* Bunge extracts T95, T70, and T40 extracted with 95%, 70%, and 40% ethanol, and the effects thereof were examined. In the same manner as in Example 3.3, LNCaP cells were treated with DHT, and *Salvia miltiorrhiza* Bunge extracts T95, T70, and T40 were administered thereto each at 5, 10, and 15 µg/ml, and then the expression of androgen receptor (AR) and prostate-specific antigen (PSA) was examined, and are shown in FIGS. 3 and 4.

As shown in FIGS. 3 and 4, each of *Salvia miltiorrhiza* Bunge extracts T95, T70, and T40 inhibited the expression of androgen receptor (AR) and prostate-specific antigen (PSA) in a dose-dependent manner, and *Salvia miltiorrhiza* Bunge extract T95 showed a most remarkable effect. In addition, *Salvia miltiorrhiza* Bunge extract T70 showed no great difference compared with T40, but showed an excellent inhibitory effect than T40. The analysis of these results based on the component contents in Table 1 confirmed that 40% to 99% ethanol, leading to remarkable increases in tanshinones extraction rates and reductions in contents of salvianolic acid and other materials, corresponded to optimal solvent concentrations.

### <Example 4: Verification of Effect of Salvia miltiorrhiza Bunge extract T in benign prostatic hyperplasia animal models>

### 4.1. Construction of testosterone-induced benign prostatic hyperplasia animal models

Sprague-Dawley rats aged 9 weeks (body weights of 350 g or less) were acclimated for one week or longer, and then used in tests. In order to induce benign prostatic hyperplasia, testicles were removed and then testosterone (dissolved in corn oil) was subcutaneously injected at a dose of 3 mg/kg once/three days for a total of 10 times.

### 4.2. Verification of Effect of Salvia miltiorrhiza Bunge extracts in benign prostatic hyperplasia animal models

Sprague-Dawley rats aged 9 weeks (body weights of 350 g or less) were randomly separated into (1) normal group, (2) benign prostatic hyperplasia induction and vehicle administration group (negative control group), (3) benign prostatic hyperplasia induction and *Salvia miltiorrhiza* Bunge extract S administration group, (4) benign prostatic hyperplasia induction and *Salvia miltiorrhiza* Bunge extract T70 administration group, (5) benign prostatic hyperplasia induction and *Salvia miltiorrhiza* Bunge extract T40 administration group, (6) benign prostatic hyperplasia induction and *Salvia miltiorrhiza* Bunge extract T10 administration group, (7) benign prostatic hyperplasia induction and saw palmetto fruit extract administration group (positive control group, certificated raw material), and (8) benign prostatic hyperplasia induction and finasteride administration group (positive control group, medicine). For the benign prostatic hyperplasia induction groups, testicles were removed and then dihydro-testosterone (dissolved in corn oil) was subcutaneously injected at a dose of 3 mg/kg once/three days for a total of 10 times, by the same method as in Example 4.1. For the normal group, an equivalent amount of corn oil was subcutaneously injected.

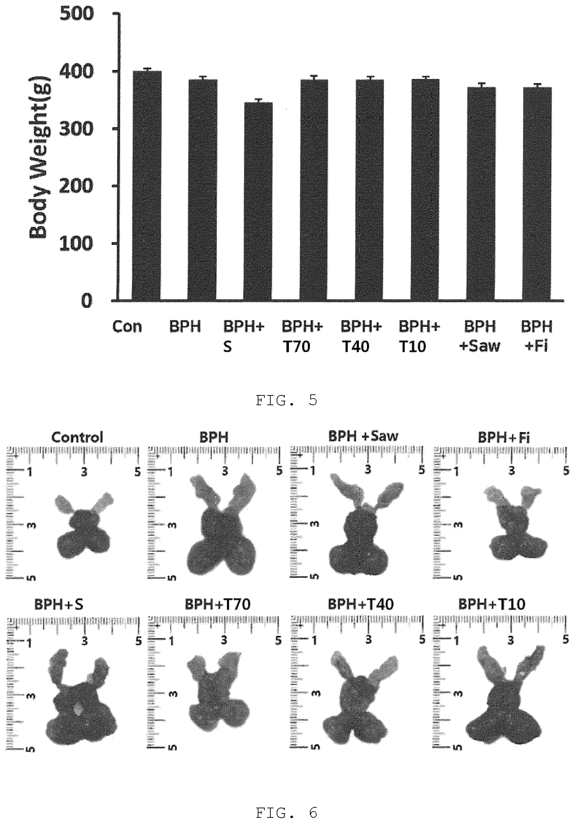

*Salvia miltiorrhiza* Bunge extracts T70 to T10 and saw palmetto fruit extract were dissolved in distilled water, and orally administered at a dose of 10-25 mg/kg once/day, for 14 days. For the finasteride administration group (Sigma, dissolved in 0.2% Tween 80), finasteride was orally administered at a dose of 10 mg/kg once/day for 14 days. For the negative control group, only distilled water was orally administered. The body weight was measured upon the end of the test, and on the next day after the ending of the last dosing and treatment, the SD-rats were sacrificed with carbon dioxide gas, and then the prostate tissue was extracted and the weight was measured. The results are shown in FIGS. 5 to 7. All animal-related procedures were discussed and approved by the Institutional Animal Care and Use Committee of the Konkuk University.

FIG. 5 shows a graph depicting body weight changes of rats by treatment with *Salvia miltiorrhiza* Bunge extracts T70 to T10 in benign prostatic hyperplasia animal models. As shown in FIG. 5, benign prostatic hyperplasia animal models (BPH) showed no significant difference in body weight compared with the control group (Con), and it was confirmed that benign prostatic hyperplasia animal models treated with testosterone after testicle removal surgery were appropriately established. The administration of saw palmetto (BPH+Saw) and finasteride (BPH+Fi) as the positive control groups showed no great difference compared with the control group (Con), and the treatment with *Salvia miltiorrhiza* Bunge extract T (BPH+*Salvia miltiorrhiza* Bunge extracts T70 to T10) also showed no significant difference in body weight. However, *Salvia miltiorrhiza* Bunge extract S showed a reduced body weight compared with the control group. *Salvia miltiorrhiza* Bunge extract S contained 15% or more of salvianolic acid B as shown in Table 1 above. According to SHAN GAO, et al. (2019), salvianolic acid B has a potent antioxidative function in cells, and thus exhibits a function of inhibiting programmed cell death initiated by an oxidation state, and besides, salvianolic acid B has been often known to have anti-inflammatory and anticancer functions. However, as reported by SHAN GAO, et al.(2019), salvianolic acid B has cytotoxicity at a predetermined concentration, and in many cases, other anticancer functions of salvianolic acid B utilize cytotoxic effects on cancer cells. The present inventors interpreted that *Salvia miltiorrhiza* Bunge extract S contains a high concentration of salvianolic acid B, and thus exhibits a predetermined level of cytotoxicity in benign prostatic hyperplasia animal models, leading to a reduction in body weight of SD rats. Especially, as shown in FIG.1 and Table 1, *Salvia miltiorrhiza* Bunge extract S predominantly contains salvianolic acid B rather showed an increase tendency in 5α reductase type 2 (5AR2) and prostate-specific antigen (PSA), which were mainly increased in benign prostatic hyperplasia, and therefore, the removal of salvianolic acid B is contemplated in order to use a *Salvia miltiorrhiza* Bunge extract in benign prostatic hyperplasia.

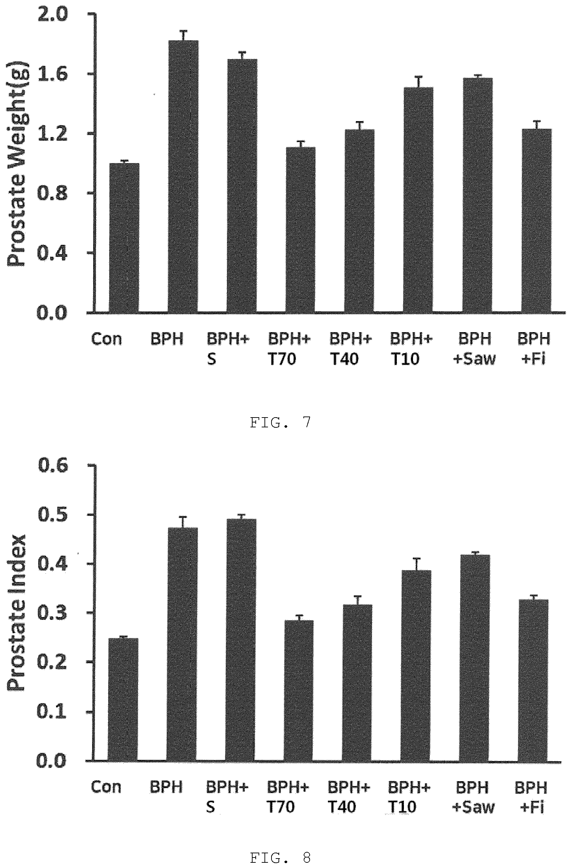

FIG. 6 shows photographs depicting prostate size changes of rats by the treatment with *Salvia miltiorrhiza* Bunge extracts T70 to T10 in benign prostatic hyperplasia animal models. The prostate sizes of rats increased by BPH treatment were reduced by the treatment with *Salvia miltiorrhiza* Bunge extracts T70 to T10. FIG. 7 is a graph showing prostate weight changes of rats by the treatment with *Salvia miltiorrhiza* Bunge extracts T70 to T10 in benign prostatic hyperplasia animal models, and FIG. 8 is a graph showing prostate index (prostate weight compared to body weight) changes of respective samples. As shown in FIGS. 6 and 8, *Salvia miltiorrhiza* Bunge extract S generally reduced the body weight of rats and hardly reduced the prostate size, and thus rather increased the prostate index (prostate weight compared to body weight) compared with benign prostatic hyperplasia models (BPH).

On the contrary, *Salvia miltiorrhiza* Bunge extracts T70 and T40 showed similar degrees of prostate weight and prostate index to the control group (Con), and the 40% ethanol extract showed almost the same effect as finasteride, which is a drug mainly used to treat benign prostatic hyperplasia or male alopecia. *Salvia miltiorrhiza* Bunge extract T10 had a certain effect on the reduction of prostate weight and prostate index, but showed no remarkable difference like *Salvia miltiorrhiza* Bunge extracts T70 and T40. It is understood that as the ethanol concentration increases, the content of tanshinones decreases and the salvianolic acid B tends to decrease, and the *Salvia miltiorrhiza* Bunge extracts extracted with 40% or higher ethanol have an inhibitory effect on androgen-related enzymes.

### 4.3. Verification of effect of Salvia miltiorrhiza Bunge extracts in prostate tissue derived from benign prostatic hyperplasia animal models

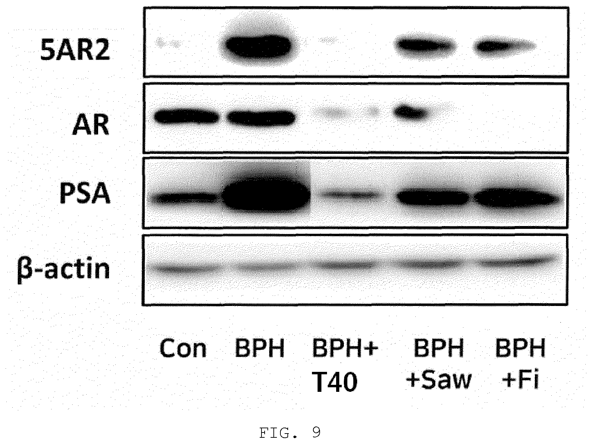

FIG. 9 shows the effect of *Salvia miltiorrhiza* Bunge extract T40 on the expression of 5α-reductase (5AR2), androgen receptor (AR), and prostate-specific antigen (PSA), which are benign prostate hyperplasia-related proteins, in benign prostate hyperplasia animal models.

The prostate tissues harvested in Example 4.2 were lysed using a cold radioimmunoprecipitation assay (RIPA) buffer containing a protease inhibitor cocktail. The lysed cells were centrifuged at 13,000 RPM for 20 minutes at 4°C, and the protein concentration was determined using BCA assay. The cell lysates (30 µg protein/sample) were separated by 10% sodium dodecyl sulfate-polyacrylamide gel electrophoresis (SDS-PAGE) at 120% for 90 minutes and transferred onto nitrocellulose membranes. The membranes were blocked with 5% skim milk at room temperature for 1 hour. Immediately afterward, primary antibodies like AR, 5AR2, PSA, and β-actin were reacted overnight to the membrane. After washed, the membranes were incubated with goat anti-rabbit IgG and anti-mouse IgG horseradish peroxidase (HRP)-conjugated secondary antibody for 1 hour at room temperature. Immunodetection was performed using an enhanced chemiluminescence (ECL) detection reagent, and the membranes were photographed using Davinch-Chemi Imaging System (Davinch-K., Seoul), and the results are shown in FIG. 9.

As shown in FIG. 9, 5AR2 and PSA significantly increased in benign prostatic hyperplasia animal models, and the protein expression of all of 5AR2, AR, and PSA was significantly inhibited by *Salvia miltiorrhiza* Bunge extract T40.

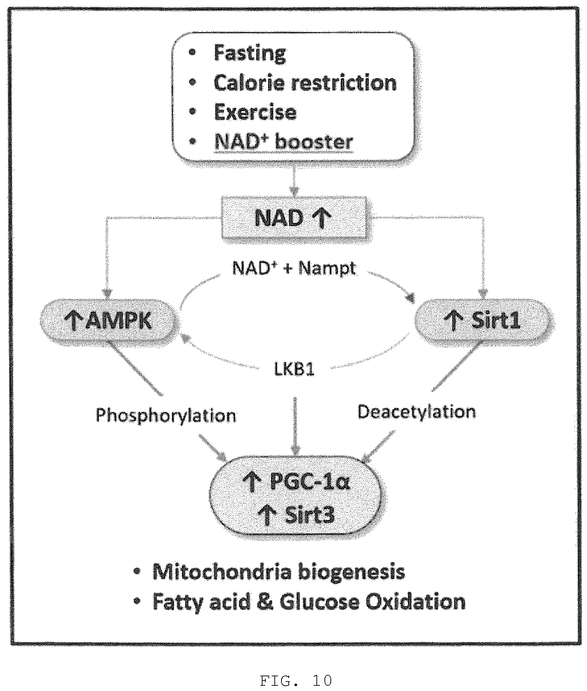

FIG. 10 is a schematic diagram showing the signaling pathway of testosterone and pharmacological mechanism of *Salvia miltiorrhiza* Bunge extracts in BPH. The cause of benign prostatic hyperplasia has not yet been clear, but aging, imbalance of male hormones (especially dihydrotestosterone (DHT)), and metabolic disorders are presumed to be main causes of benign prostatic hyperplasia. Currently used saw palmetto fruit extract (a health-functional food certificated material) and finasteride (benign prostate hyperplasia treatment) target male hormones only, but for the maximization of treatment effects, strategies capable of improving aging, male hormones in unbalanced states, and metabolic disorders are needed. A main tanshinone component of the ethanol extract of *Salvia miltiorrhiza* Bunge, which is an NAD+ booster, and targets "NQO1" enzyme in the cytoplasm and converts the co-substrate NADH into NAD+, thereby increasing the concentration of NAD+ *in vivo.* The NQO1 enzyme is 2-electron reductase of the flavoprotein family, and reduces quinone bases into hydroquinone by using NADH or NADPH as a cofactor, and generally removes reactive oxygen species and performs vitamin K metabolism. An increase in intracellular NAD+ by a NAD+ booster (main tanshinone component) activates sirtuin, AMPK, and PGC-1α, which simultaneously enhances both the amount and function of mitochondria, thereby activating energy metabolism in cells. In particular, sirtuin is a protein having an anti-aging effect; and AMPK and PGC-1α are proteins that play a core role in the improvement of metabolic diseases. These proteins were expected to have a high potential to effectively relieve "benign prostatic hyperplasia" caused by "aging, metabolic diseases, and male hormone imbalance", and it was verified through the above results that the 40% or higher (40-99%) ethanol extracts of *Salvia miltiorrhiza* Bunge had an excellent effect on the prevention or treatment of benign prostatic hyperplasia or alopecia.

## Claims

1. A composition comprising a *Salvia miltiorrhiza* Bunge extract as an active ingredient for treatment or prevention of benign prostatic hyperplasia or alopecia.

2. The composition of claim 1, wherein the *Salvia miltiorrhiza* Bunge extract includes an extract obtained by subjecting *Salvia miltiorrhiza* Bunge to extraction with water, an alcohol having one to six carbon atoms, or a mixture solvent thereof.

3. The composition of claim 1, wherein the *Salvia miltiorrhiza* Bunge extract is a 40-99% ethanol extract.

4. The composition of claim 1, wherein tanshinones are contained in 2-70% of the total weight of the *Salvia miltiorrhiza* Bunge extract.

5. The composition of claim 4, wherein the tanshinones include, as effective components, one or more of tanshinone1, tanshinone2a, cryptotanshinone, 15,16-dihydrotanshinone, and tanshinoic acid.

6. The composition of claim 4, wherein the tanshinones include 15,16-dihydrotanshinone as an effective component.

7. The composition of claim 1, wherein the *Salvia miltiorrhiza* Bunge extract reduces the expression of at least one protein of the group consisting of 5α-reductase type 2 (5AR2), prostate-specific antigen (PSA), steroid receptor co-activator-1 (SRC-1), and androgen receptor (AR).

8. The composition of any one of claims 1 to 7, wherein the *Salvia miltiorrhiza* Bunge extract is prepared by a method for preparing a *Salvia miltiorrhiza* Bunge extract, the method comprising:
(1) crushing *Salvia miltiorrhiza* Bunge with impurities removed therefrom;
(2) immersing the *Salvia miltiorrhiza* Bunge crushed in step (1) in 40-99% ethanol (EtOH) at 50-80°C, followed by standing for 2-24 hours;
(3) separating the ethanol after the extraction from *Salvia miltiorrhiza* Bunge in step (2), and adding new ethanol to residues to immerse the residues in a 40-99% ethanol (EtOH) at 50-80°C, followed by standing for 2-12 hours;
(4) separating the ethanol after the extraction from *Salvia miltiorrhiza* Bunge in step (3), and adding new ethanol to residues to immerse the residues in a 40-99% ethanol (EtOH) at 50-80°C, followed by standing for 2-5 hours;
(5) collecting the ethanol separated in each of steps (2) to (4), followed by evaporation and drying at 45-75°C; and
(6) grinding a solid extract obtained from the evaporation and drying in step (5), followed by filtration at 80 mesh or greater, to obtain the *Salvia miltiorrhiza* Bunge extract.

9. The composition of claim 8, wherein in step (1) of crushing *Salvia miltiorrhiza* Bunge with impurities removed therefrom, *Salvia miltiorrhiza* Bunge is ground by cryogenic micro grinding technology (CMGT) in which *Salvia miltiorrhiza* Bunge is frozen at a cryogenic temperature of -196 to -80°C for a short time and ground to 5 mm or less.

10. A health functional food comprising a *Salvia miltiorrhiza* Bunge extract as an active ingredient for prevention or alleviation of benign prostatic hyperplasia or alopecia.

11. The health functional food of claim 10, wherein the *Salvia miltiorrhiza* Bunge extract includes an extract obtained by subjecting *Salvia miltiorrhiza* Bunge to extraction with water, an alcohol having one to six carbon atoms, or a mixture solvent thereof.

12. The health functional food of claim 11, wherein the alcohol is 40-99% ethanol.
